Europäisches Patentamt

European Patent Office

Office européen des brevets

(19)

(11) Publication number: **0 159 446**
**A2**

(12) **EUROPEAN PATENT APPLICATION**

(21) Application number: **84308425.2**

(22) Date of filing: **04.12.84**

(51) Int. Cl.⁴: **A 61 H 39/00,** A 61 N 5/06

---

(30) Priority: **09.04.84 AU 4482/84**

(43) Date of publication of application: **30.10.85** Bulletin 85/44

(84) Designated Contracting States: **AT BE CH DE FR GB IT LI LU NL SE**

(71) Applicant: **Lasercare (Australia) Pty. Limited, 203-233 New South Head Road, Edgecliffe New South Wales 2027 (AU)**

(72) Inventor: **Chapman, Carolin, 114 Fairy Princes Highway, Fairy Meadow New South Wales 2519 (AU)**

(74) Representative: **Bannerman, David Gardner et al, Withers & Rogers 4 Dyer's Buildings Holborn, London, EC1N 2JT (GB)**

(54) **Cosmetic scalp treatment.**

(57)     A method of treating the scalp to promote hair growth which comprises bathing the scalp with the output from a cosmetic/medical laser for periods in the range of ten minutes and one hour, and preferably between twenty minutes and forty minutes, the periods being spaced from a few days to several weeks and, preferably, also specifically, applying the laser to acupuncture points which are directly related to hair growth.

EP 0 159 446 A2

## COSMETIC SCALP TREATMENT

This invention relates to a cosmetic scalp treatment and, more particularly, to a method of treatment whereby the growth of hair can be stimulated even, in many cases, where a person is effectively bald.

Hair loss is a by-product of the natural ageing process and is more marked in some individuals than in others.

Wigs and hairpieces have been employed for many years in order to conceal such hair loss, presumably because the wearers have believed that their personal appearance was thereby improved.

Hair transplants have also been carried out in an attempt to combat the effect of hair loss, and have achieved some significance in the cosmetic field.

The mechanism of hair loss and hair regrowth is not fully understood, but it is believed that normally the hair follicles are located some distance below the surface of the scalp but when a person is losing hair the follicles tend to move to the surface of the scalp and, in so moving, tend to move from the normal source of blood supply. Consequentially a hair root located in the follicle is also starved of blood supply and, if it should grow, tends to be weak. In a person suffering hair loss, if the follicles could be caused to move to the normal position, it is believed that

0159446

it is possible that the blood supply would be re-established. Further, in most persons, there are a large number of quiescent hair roots in follicles and if the growth of these could be activated, there would be a regrowth of hair.

The object of the invention is to provide a cosmetic treatment for the scalp which causes growth of hair.

The invention, in its broadest sense, comprises a method of treating the scalp cosmetically to promote hair growth comprising bathing the scalp with the output from a cosmetic/medical laser for periods in the range of ten minutes and one hour at time periods spaced from a few days to several weeks to thereby stimulate the growth of hair on the scalp.

The preferred period of bathing may be from twenty to forty minutes.

Preferably, in the method, the scalp is specifically stimulated at the acupuncture points thereon.

More specifically, the method may include the stimulation of acupuncture points away from the scalp which have to do with hair growth.

It is also perferred that the treatment be combined with other beneficial processes, such as regular scalp massage, vitamim supplementation and proper diet.

In order that the invention may be more readily understood, we shall describe an embodiment of the operation of the process of the invention and summarise results so far achieved.

Firstly, as was stated above, the actual mechanism by which hair loss and growth is effected is not fully understood and, as a consequence of this, the manner in which the invention effects its purpose is not understood, but it appears to be by the revitalisation of hair roots which have been dormant and this is possibly because of an increase in the effectiveness of the blood circulation in the area adjacent the follicles in which the roots are located.

In the treatment we use a 1 mW helium neon laser, which is a known machine for treating the complexion and which has approval by the interested Government Departments and Authorities.  A normal treatment takes approximately 30 minutes.

During the first ten to fifteen minutes of this period, we use the laser to stimulate acupuncture points which are recognised as being associated with the scalp and we may stimulate more than 30 of these, each for a very short period.  Whilst many of these points are on the scalp itself, some of the points are spaced therefrom.  Laser machines are known which, when moved over the body, give a reading, or a changed reading, when located on an acupuncture point and this, provided the operator knows where the points should be located, permits very accurate location of the points.

The specific location of these acupuncture points will not be described further herein, as they are known in the art.

Subsequently to stimulating these points, we then provide a general bathing of the head dome for a period of some fifteen minutes.

Subsequent to this we provide a scalp massage and suggest that the client massages the scalp regularly between treatments as such massage helps to tone the skin.

A course of treatment can comprise some fifteen such treatments at fortnightly intervals and it is presently proposed to provide a booster treatment each three months.

We have found that, when a client is being treated, the growth pattern is that the growth starts from the edge of the bald area and moves towards the centre of the head or towards the front of the head, depending upon the manner in which the baldness presents.

Generally, we find that the regrowing hair normally presents as very fine hair which thickens as growth continues.

As the process has been developed only recently, we are not sure of the optimum periods between treatments nor are we sure as to the optimum maintenance program after regrowth has been consolidated.

It does appear that maintenance treatment approximately once every three months gives satisfactory results, but it may be that less regular treatments would also be satisfactory.

In order to best describe the efficiency of the process, reference will be made to qualitative results which have been obtained from Centres applying the invention in Wollongong and Edgecliff in New South Wales.

As the operation of the method has only been carried out over a relatively short period, it is not possible, at the present time, to give full quantitative results of the method.

<u>Wollongong</u> – approximately three hundred and fifty people have been treated by the method and, of these, 80% have been males and 20% females.

The general responses of patients, in age groups are:

> 18-25 years – some regrowth has been sighted after the third to fifth treatments, the treatments being effected fortnightly.

> The initial regrowth is in the form of very fine, infantile, hair but, as the regrowth continues, the hair tends to thicken to a normal hair and tends to exhibit the characteristics of normal hair, if any, growing in the surrounding area.

Of those in this age group who have had a full series of fifteen treatments, or who are continuing with the treatment and have had more than five treatments, we have found that there is a positive response in 90% of all cases and the regrowth in the crown area has been effectively to 75% of what would be considered normal and, in the front and sides, 50%.

25-45 years - positive results occur, on average, between the fourth and sixth treatments, although some respond earlier than this.

In this age group the regrowth in the crown area has been shown to be not as substantial as in the younger age group but has been approximately 50% and the regrowth in the front and sides area has also been approximately 50%, that is effectively the same as for the younger age group.

45-65 years - somewhat surprisingly, positive results have been occurring in this age group at between the second and fourth treatments, that is, on average, somewhat earlier than in the middle age group.

In this age group, over a series of fifteen or more treatments, there has been overall a positive result in 90% of cases and in clients, in their late fifties, there has been a 25% to 50% regrowth on average.

In a particular case in this sample, one client who had been bald for thirty years has had a 50% to 75% regrowth of hair.

We have also found that women in the later age group, 40-55 years, appear to respond quicker than men and, on average, recognisable changes occur between the second and third treatments and the growth is more even over the head whereas, with men, there tends to be a higher growth in the crown area than the front and sides.

Edgecliff - the number of clients treated in Edgecliff has been approximately one hundred and fifty. Of these, 75% are males and 25% are females.

Generally it has been noted that the younger the client and the shorter the time of hair falling or balding, the better the results which have been achieved, although, in most cases, between the third and sixth treatment there as positive signs of hair

0159446

growth.

Where the client has previously had a hair transplant, the time before regrowth is noticed tends to be longer than in the case of persons who have not had hair transplants but there has been positive growth both in the plugs and in the surrounding scalp.

Also, we have noted that men appear to respond more positively than women to the treatment although, with some women, we have had extremely satisfactory results.

The following table is an indication of the results for a number of individual clients:-

| CLIENT | NO. OF TREATMENTS | AGE | SEX | RESULTS |
|---|---|---|---|---|
| J.A. | 8 | 24 | M | No longer falling after fourth treatment. Fine blonde regrowth around seventh treatment. |
| P.A. | 9 | 29 | M | New hair visible after sixth treatment. People commenting in increasing amount and thickness of hair, both at front, around plugs and at crown. |
| T.R. | 3 | 34 | M | Stated at third treatment he was aware of regrowth. |
| D.B. | 9 | 60 | F | Hair stopped falling around third treatment. Hair longer and thicker and a severe scalp condition she had been suffering from for many years has improved considerably. |
| W.S. | 9 | 37 | M | Noticing new hair around plugs at front and on top around seventh treatment. |
| R.W. | 9 | 38 | M | Hair fall ceased after fourth treatment and general condition of hair much improved by sixth treatment. New hair was noticeable particularly around crown. |
| J.M. | 7 | 49 | M | No falling hair after third treatment. Obvious new growth after fifth treatment. |
| A.G. | 7 | 37 | F | No falling hair after fourth |

treatment.    Some fine new growth after sixth treatment.

| | | | | |
|---|---|---|---|---|
| W.C. | 4 | 37 | M | Falling slowed after third treatment. |
| I.P. | 5 | 39 | F | Definite thickening, lengthening of hair after third treatment.   New growth visible after fourth treatment. |
| N.H. | 9 | 76 | M | New growth after 6th treatment.  New hair clearly visible on top mid section of scalp. |
| J.R. | 6 | 17 | M | Hair stopped falling after fourth treatment. |
| J.L. | 7 | 25 | M | Fine blonde hairs growing in front after fifth treatment. |
| J.S. | 8 | 22 | M | Noticing new dark growth at front following fifth treatment. |
| P.M. | 7 | 32 | M | Noticing lot of new dark hair all over scalp after fifth treatment. |
| C.S. | 10 | 56 | M | Hair fall stopped after second treatment.  New hair over front and crown after fourth treatment.  General thickening of all hair. |
| W.McM | 7 | 19 | M | Hair fall stopped after second treatment.  New growth after fourth treatment. |
| R.W. | 7 | 50 | M | Hair fall stopped after third treatment.  New hair in frontal region after fifth treatment. |
| M.B. | 7 | 23 | M | Obvious signs of new growth at front after fourth treatment. |
| P.McG | 5 | 40 | M | Significant decrease in hair falling after third treatment. |
| S.R. | 8 | 33 | M | Hair stopped falling around third treatment.  New dark growth around sixth treatment. |
| W.P. | 9 | 41 | M | Many new dark hairs appearing all over scalp and around hair transplant plugs after sixth treatment. |
| R.K. | 7 | 31 | M | Hair fall ceased after third treatment.   New growth, particularly |

around   crown   after   fifth-sixth treatment.

| K.McT | 10 | 53 | M | New hair after sixth treatment and considerable regrowth by tenth treatment. |
|-------|----|----|---|------------------------------------------------------------------------------|
| B.G.  | 11 | 39 | M | When client commenced program had no hair on top of head at all. New hair after third treatment and by eleventh treatment both thick and fine dark hairs all over scalp, longest being about one inch in length. |

CLAIMS:                                                    0159446

1.   A method of treating the scalp cosmetically to promote hair growth comprising bathing the scalp with the output from a cosmetic/medical laser for periods in the range of ten minutes and one hour at time periods spaced from a few days to several weeks to thereby stimulate the growth of hair on the scalp.

2.   A method as claimed in claim 1 wherein the period of bathing lies in the range of twenty minutes to forty minutes.

3.   A method as claimed in claim 1 or claim 2 wherein the method includes specifically stimulating the acupuncture points on the scalp with the laser before, during or after the bathing of the scalp with the laser.

4.   A method as claimed in any one of claims 1 to 3 wherein acupuncture points which have a relationship with the scalp, but which are not on the scalp, are also bathed during the treatment.

5.   A method as claimed in any preceding claim wherein the laser has an output of the order of 1mW.

0159446

6.    A method as claimed in any preceding claim which includes massaging of the scalp.